(19) Europäisches Patentamt
European Patent Office
Office européen des brevets

(11) Numéro de publication: 0 132 204
B1

(12) # FASCICULE DE BREVET EUROPÉEN

(45) Date de publication du fascicule du brevet: 12.07.89

(51) Int. Cl.⁴: B 03 D 1/00, C 07 C 153/05

(21) Numéro de dépôt: 84401501.6

(22) Date de dépôt: 17.07.84

(54) **Nouveaux thioamides, leurs préparation et applications.**

(30) Priorité: 19.07.83 FR 8311880

(43) Date de publication de la demande: 23.01.85 Bulletin 85/4

(45) Mention de la délivrance du brevet: 12.07.89 Bulletin 89/28

(84) Etats contractants désignés: DE SE

(56) Documents cité:
EP-A-0 059 596
EP-A-0 070 088
FR-A-1 501 846
FR-A-2 429 617
US-A-3 994 889

(73) Titulaire: SOCIETE NATIONALE ELF AQUITAINE (PRODUCTION), Tour Aquitaine, F-92400 Courbevoie (FR)

(72) Inventeur: Levesque, Guy, rue d'Ardennes Contest, F-14000 Caen (FR)
Inventeur: Tozzolino, Pierre, Chemin Rural Carrerot Serres- Morlaas, F-64160 Morlaas (FR)

(74) Mandataire: Kohn, Armand, 5 Avenue Foch, F-92380 Garches (FR)

EP 0 132 204 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention se rapporte à des thioamides porteurs d'une seconde fonction. Elle comprend l'utilisation de tels amides en tant que collecteurs dans la flottation de minerais. L'invention concerne également certains nouveaux thioamides, portant une seconde fonction, ainsi qu'un procédé de leur préparation.

Des thioamides ont différentes applications industrielles, par exemple en tant qu'adjuvants des compositions cosmétiques, pharmaceutiques, améliorants des huiles de graissage, fongicides, accélérateurs de vulcanisation, etc.; ils peuvent également être utilisés comme collecteurs dans la flottation de minerais. Il apparaît que la présence d'une fonction autre qu'amide, dans la molécule, procure des propriétés intéressantes à celle-ci; c'est le cas notamment des groupes tels que -OH, $-NH_2$ ou -COOH. Cependant, la préparation de tels composés bifonctionnels, connue jusqu'à présent, n'est pas très pratique; on sait que cette méthode connue, qui consiste à faire réagir du formaldéhyde sur un thioamide du type $R\text{-}CS\text{-}NH_2$, pour donner le dérivé $R\text{-}CS\text{-}NH\text{-}CH_2OH$ (H. BOEHME, H.H. HORTZEL, Archiv. Pharm. vol. 300, 1967, p. 241) donne des rendements qui laissent à désirer et exige déjà la possession d'un thioamide, matière première relativement coûteuse. De plus, ce procédé antérieur permet seulement l'obtention de thioamides portant l'hydroxyle, alors que des composés renfermant d'autres groupes fonctionnels, comme $-NH_2$, -COOH, -SH, -CN, sont intéressants.

L'invention concerne un procédé de flottation de minerais, dans lequel un thioamide est employé en tant que collecteur, caractérisé en ce que ce thioamide porte un second groupe fonctionnel et répond à la formule

```
R-C-NH-R''Z ou  R-C-N(R''Z)2
  ||              ||
  S               S
```

où

R est un alkyle, alkényle, cycloalkyle, cycloalkényle ou aryle, éventuellement substitué,

R'' est un alkylène, alkénylène, cycloalkylène, cycloalkénylène ou arylène, éventuellement substitué comme R ou différemment, et

Z, le second groupe fonctionnel en plus de celui du groupe amide, est -OH, $-NH_2$ ou -COOH, dont les H peuvent être substitués par un radical hydrocarboné, un groupe d'amine cyclique ou un groupe $-HC(OCH_3)_2$.

La présente invention apporte également un procédé qui permet l'obtention d'un grand nombre de divers thioamides, dans des conditions bien économiques, à l'état de pureté très satisfaisant; ce procédé utilise des matières premières facilement accessibles industriellement.

Le procédé, suivant l'invention, consiste à faire réagir un mono- ou dithio-ester sur une amine dont un ou plusieurs atomes de carbone portent une seconde fonction (Z). Cette dernière peut être notamment -OH, $-NH_2$, -COOH, -CN, -SH, etc. Dans chacun de ces groupes fonctionnels, un ou plusieurs H peuvent être substitués par des groupes hydrocarbonés. Lorsque la molécule porte plusieurs de telles fonctions, elles peuvent être semblables ou différentes.

Le groupe fonctionnel Z peut se présenter sous la forme substituée, telle que $-NHR^3$, $-NR^3R^3$, $-OR^3$, $-COOR^3$, etc., où $R^3$ est un radical hydrocarboné, le plus souvent un alkyle inférieur. Il est également à noter que la fonction amine Z peut se trouver à l'état d'une amine cyclique, par exemple pyridine, pipérazine, pyrimidine, pyrrole, triazole, tétrazole, acide aminé ou similaires.

La réaction, corresopndant au procédé suivant l'invention, peut être représentée comme suit:

```
R-C-SR'  + NH2 - R''Z →  R-C-NH-R''Z + R'SH
  ||                       ||
  S                        S
```

ou bien

```
R-C-SR'  + NH-(R''Z)2 →  R-C-N(R''Z)2 + R'SH
  ||                       ||
  S                        S
```

où -SR' peut être remplacé par -OR'.

Dans une variante, l'ester de départ peut être un monothioester

```
R-C-OR',
  ||
  S
```

auquel cas le composé éliminé est l'alcool R'OH à la place du mercaptan R'SH.

Le procédé est applicable à un grand nombre de thioesters, c'est-à-dire que R peut être constitué par des

groupes ou chaînes très divers; ils peuvent être notamment des alkényles, alkyles, cycloalkyles, cycloalkényles, aryles, éventuellement substitués, ce qui entend, entre autres, des alkaryles ou aralkyles.

Le radical R' du thioester peut également prendre différentes formes, alkyles, alkényles, aryles, etc., mais, tant pour des raisons économiques que pour celles de l'empêchement stérique, il y a intérêt à ce que ce groupe soit aussi simple que possible, ce qui revient pratiquement à utiliser surtout les alkyles inférieurs, et principalement $-CH_3$ et $C_2H_5$. Le radical ou chaîne R'', dans l'amine utilisée, peut être choisi parmi ceux qui sont cités plus haut, au sujet de thioamides utilisés à la flottation; les plus courants sont des chaînes aliphatiques notamment des polyméthylènes c'est-à-dire $-(CH_2)_n-$, n étant de préférence 1 à 18.

Le mode opératoire préféré consiste à dissoudre le thioester dans un solvant approprié qui peut être un hydrocarbure chloré, comme par exemple dichlorométhane, dichloroéthane, trichloroéthane, trichloroéthylène, tétrahydrofuranne ou autre solvant inerte vis-à-vis des réactifs en présence; à la solution obtenue, on ajoute l'amine choisie, éventuellement en léger excès. On laisse la réaction se poursuivre pendant le temps nécessaire variable avec la nature des réactifs,mais le plus souvent compris entre 1/2 h et 6 h. La température est de 0 à 50°C et, de préférence, entre 0 et 25°C, sauf lorsque R est un aryle halogéné, auquel cas la température préférée est de l'ordre de 40°C.

Suivant ce procédé, on a préparé, dans le cadre de la présente invention, une série de composés nouveaux, constitués par des thioamides pouvant être représentés par la formule:

R-C-NH-R''Z ou R-C-N(R''Z)$_2$
‖ ‖
S S

où R est une chaîne alkylique, R'' est une chaîne $-(CH_2)_n-$, n étant 1 à 6, ou bien

-CH-,
|
$CH_3$

tandis que Z représente -OH, -COOH l'atome de H pouvant y être remplacé par un substituant, notamment un alkyle. Plus particulièrement R est un alkyle en $C_7$ à $C_{11}$.

Dans les exemples non limitatifs, qui suivent, on décrit la préparation de quelques-uns des produits définis plus haut. Les propriétés physiques de ces composés sont réunies dans un tableau à la fin des exemples. Ces thioamides et leurs homologues sont utilisables avec succès pour la séparation de minerais, surtout sulfureux, par flottation.

**Exemples 1 à 4**

Synthèse de N-(hydroxyl-2 éthyl)thiocarbamide

On prépare d'abord une solution de 0,5 mole de dithioate d'éthyle $R-CS-SC_2H_5$ (dont le R est indiqué plus bas) dans 100 ml de dichlorométhane. 0,5 mole d'éthanolamine $NH_2CH_2CH_2OH$ sont ajoutés sous agitation à la solution obtenue. Le mélange est laissé au repos à 25°C, pendant 3 h. Ensuite, le solvant est évaporé et le résidu est cristallisé, puis repurifié par recristallisation dans l'hexane additionné d'une trace de solvant polaire, notamment de dichlorométhane.

Avec ce mode opératoire, on a préparé:

Ex. 1: R étant $-C_{11}H_{23}$, du N-(hydroxy-2 éthyl)thiododécanamide
$CH_3(CH_2)_{10}CS-NH-CH_2CH_2OH$
avec un rendement de 86 %.

Ex. 2: R étant $C_6H_5$, du N-(hydroxy-2 éthyl)thiobenzamide
$C_6H_5-CS-NH-CH_2CH_2OH$
avec un rendement de 45 %.

Ex. 3: R étant $CH_3O-C_6H_4$, du N-(hydroxy-2 éthyl)méthoxy-4 thiobenzamide
$CH_3O-C_6H_4-CS-NH-CH_2CH_2OH$
avec un rendement de 84 %.

Ex. 4: R étant $CH_3C_6H_4$, du N-(hydroxy-2 éthyl) méthyl-4 thiobenzamide
$CH_3-C_6H_4-CS-NH-CH_2CH_2OH$
avec un rendement de 69 %.

**Exemple 5**

Préparation de N-(amino-3-propyl)thio-dodécanamide

10,4 g de dodécane-dithioate d'éthyle sont dissous dans 100 ml de toluène, et la solution obtenue est mélangée avec une solution de 30 g de diamino-1,3 propane dans 200 ml de toluène. Le mélange est maintenu, avec agitation, à environ 10° C, pendant 1 h.

Le toluène est ensuite distillé sous pression réduite; l'excès d'amine est éliminé par distillation.

Après recristallisation du résidu dans de l'hexane, on obtient, avec un rendement de 45 %, du N-(amino-3 propyl)dodécane-thioamide pur, $CH_3(CH_2)_{10}$-CS-NH-$CH_2CH_2CH_2NH_2$.

**Exemples 6 à 10**

Préparation de N-(amino-6 héxyl)thioamides

Le mode opératoire consiste à ajouter 0,5 mole de dithioate d'alkyle R-CS-SR' choisi, dilué par 100 ml de toluène, à 200 ml de solution toluénique de 0,5 mole de diamino-1,6 hexane.

La disparition de la couleur du thioester permet de suivre la réaction; celle-ci est généralement terminée après 30 minutes à 25° C. Exceptionnellement, dans le cas de l'exemple 9, il a fallu 1 heure à 40° C. Le toluène et la majeure partie de l'excès d'amine sont éliminés par distillation sous pression réduite, pour être réutilisés dans une nouvelle préparation, après séparation du mercaptan formé. Le résidu de la distillation est lavé avec une petite quantité d'eau qui achève l'élimination de l'amine restante.

Pour les composés ci-dessous, on a redissous le résidu dans du toluène et on a fait passer du gaz HCl sec dans la solution obtenue, faisant ainsi précipiter le chlorhydrate du thioamide formé.

| Ex. n° | Thioester utilisé | Thioamide obtenu | Rendement % sur le thioester |
|---|---|---|---|
| 6 | Décane dithiotate d'éthyle | $C_9H_{19}CS$-$NH(CH_2)_6NH_2$.HCl | 91 |
| 7 | Benzène-dithiotate de méthyle | $C_6H_5CS$-$NH(CH_2)_6NH_2$.HCl | 62 |
| 8 | Para-méthoxybenzène-dithioate d'éthyle | $CH_3$ (para)<br>\|<br>O<br>\|<br>$C_6H_4CS$-$NH(CH_2)_6NH_2$.HCl | 64 |
| 9 | Para-chlorobenzène dithioate d'éthyle | Cl (para)<br>\|<br>$C_6H_4CS$-$NH(CH_2)_6NH_2$.HCl | 25 |
| 10 | Para-méthyl benzène dithiotate de méthyle | $CH_3$<br>\|<br>$C_6H_4CS$-$NH(CH_2)_6NH_2$.HCl | 67 |

Des résultats similaires sont obtenus à partir d'esters mono-thioïques correspondants: R-CS-OR'.

**Exemples 11 et 12**

Thioamides de N-(diméthylamino-2 éthane)

Le mode opératoire des exemples précédents est appliqué à la N,N-diméthyl éthylène diamine $(CH_3)_2N$-$CH_2CH_2$-$NH_2$ que l'on fait réagir avec un O-alkyle thionester R-CS-OR'.

11. Le thionester employé, décane-thioate d'éthyle, $CH_3(CH_2)_8CS$-$OC_2H_5$, a donné du N-(diméthyl-amino-2 éthyl)-thiodécanamide,

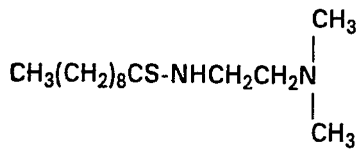

avec un rendement de 81 %.

Même résultat est obtenu à partir du décane dithioate d'éthyle $CH_3(CH_2)_8CS\text{-}SC_2H_5$.

12. A partir du para-chlorobenzène dithioate d'éthyle $Cl\text{-}C_6H_4CS\text{-}SC_2H_5$, on obtient le N-(diméthyl-amino-2 éthyl)chloro-4 thiobenzamide.

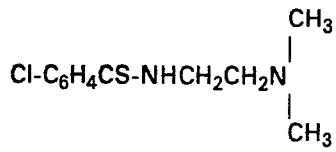

le rendement étant 68 %.

**Exemples 13 et 14**

Obtention de N-(diméthoxy-2,2 éthyl)thioamides

On opère comme dans les exemples 1 à 4, mais l'éthanolamine est remplacée par la diméthoxy-2,2 éthylamine

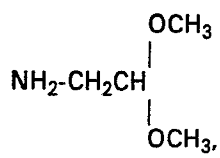

ce qui conduit à des thioamides porteuses de fonctions acétal, comme suit.

13. En partant de l'ester éthylique de l'acide décanethioïque $C_9H_{19}CS\text{-}OC_2H_5$, on obtient ainsi, avec un rendement de 69 %, du N-(diméthoxy-2,2 éthyl-)thiodécanamide,

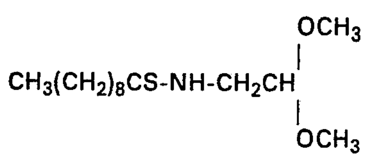

14. A partir du p. chloro-benzène dithioate d'éthyle, on a le N-(diméthoxy-2,2 éthyl)chloro-4 thiobenzamide

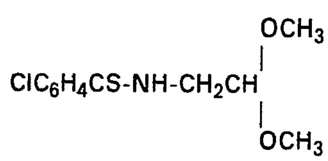

avec un rendement de 57 %.

**Exemple 15**

Préparation d'un thioamide à fonction carboxylique

L'amine, utilisée ici, est un acide aminé. A 300 ml de solution aqueuse à 10 % de NaOH, ayant dissous 0,2 mole d'octane dithioate de méthyle $CH_3(CH_2)_6CS\text{-}SCH_3$, on ajoute par petites portions 0,2 mole de D.L.-alanine,

$NH_2$-CH-COOH
|
$CH_3$

Le mélange est maintenu à la température ambiante pendant 15 heures. On acidifie ensuite avec 300 ml d'acide aqueux à 20 % HCl, ce qui fait précipiter le thioamide formé. Ce dernier est purifié par recristallisation dans de l'hexane renfermant un peu d'éther.

Le N-(carboxy-2 éthyl)thio-octamide,

$CH_3(CH_2)_6$CS-NH-CH-COOH
|
$CH_3$

est ainsi obtenu avec un rendement de 76 % sur le thioester mis en oeuvre.

**Exemples 16 à 18**

Thioamides porteurs d'un groupe pyridyle

Les dithioesters choisis sont mis à réagir avec de l'aminométhyl-2 pyridine (ou amino α-picoline)

FIG00/18

suivant la technique des exemples 1 à 4.

Les dithioesters utilisés étant respectivement ceux des exemples 8, 9 et 10, indiqués plus haut, on obtient les thioamides suivants.

16. N-(pyridyl-2 méthyl)méthoxy-4 thiobenzamide

$CH_3OC_6H_4$CS-NH-$CH_2$-FIGFIG

avec 60 % de rendement.

17. N-(pyridyl-2 méthyl)chloro-4 thiobenzamide, avec un rendement de 54 %.
18. N-(pyridyl-2 méthyl)méthyl-4 thiobenzamide, avec un rendement de 56 %.

**Exemple 19**

Préparation d'un thioamide portant sur l'azote deux substituants hydroxylés, notamment de N-bis(hydroxy-2 éthyl)

$CH_2CH_2OH$
|
dodécane-thioamide, $CH_3(CH_2)_{10}$CS-N
|
$CH_2CH_2OH$

Le mélange de 0,2 mole de dodécane dithioate d'éthyle avec 0,24 mole de diéthanolamine est chauffé à 160°C pendant 30 minutes. Après refroidissement, on ajoute un égal volume d'eau; le tout est encore refroidi jusqu'à séparation d'une huile; ce traitement est renouvelé pour éliminer l'excès de diéthanolamine.

Le thioamide obtenu, dont la formule est donnée ci-dessus, est une huile légèrement soluble dans l'eau.

Des conditions opératoires semblables sont à utiliser avec d'autres dialkanolamines.

**Exemple 20**

Production d'un thioamide ayant un groupe carboxylé sur l'azote

On dissout 18 g d'acide amino-2 propionique (β-alanine) dans 100 ml de soude aqueuse à 10 %; on ajoute ensuite 21 g d'octane dithioate d'éthyle dans 200 ml de tétrahydrofuranne; le mélange est agité 48 heures, puis le solvant organique distillé. Après refroidissement, on acidifie par 200 ml d'acide chlorhydrique à 20 %; le précipité formé est séparé, puis lavé à l'HCl à 1 % et à l'eau.

Après séchage, on peut recristalliser dans du toluène.

$CH_3-(CH_2)_6-CS-NH-CH_2CH_2-CO_2H$
Rendement 73 %

**Temperatures de fusion de thioamides** R-C-NH-R''Z (C=S)

| Ex. n° | R | R'' | Z | F°C |
|---|---|---|---|---|
| 1 | $CH_3(CH_2)_{10}-$ | $-CH_2CH_2-$ | -OH | 53 |
| 2 | $C_6H_5-$ | " | " | 94 |
| 3 | $CH_3OC_6H_4-$ | " | " | 97 |
| 4 | $CH_3C_6H_4-$ | $-CH_2CH_2-$ | -OH | 122 |
| 5 | $CH_3(CH_2)_{10}-$ | $-CH_2CH_2CH_2-$ | $-NH_2$ | 104 |
| 6 | $CH_3(CH_2)_8-$ | $-(CH_2)_6-$ | $-NH_2.HCl$ | 168 |
| 7 | $C_6H_5-$ | " | " | 125 |
| 8 | p. $CH_3OC_6H_4-$ | " | " | 179 |
| 9 | p. $Cl-C_6H_4$ | " | " | 173 |
| 10 | p. $CH_3-C_6H_4$ | " | " | 155 |
| 11 | $CH_3(CH_2)_8-$ | $-CH_2CH_2-$ | $CH_3$<br>\|<br>-N<br>\|<br>$CH_3$ | 38 |
| 12 | p. $Cl-C_6H_4-$ | " | " | 82 |
| 13 | $CH_3(CH_2)_8-$ | $-CH_2$ | $OCH_3$<br>\|<br>-CH<br>\|<br>$OCH_3$ | 22 |
| 14 | p. $Cl-C_6H_4-$ | " | " | 34 |
| 15 | $CH_3(CH_2)_6-$ | -CH-<br>\|<br>$CH_3$ | -COOH | Décomposition |
| 16 | p. $CH_3OC_6H_4$ | $-CH_2-$ | 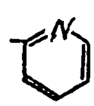 | 94 |
| 17 | p. $Cl-C_6H_4-$ | $-CH_2-$ | 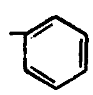 | 106 |
| 18 | p. $CH_3-C_6H_4-$ | " | " | 81 |
| 20 | $CH_3(CH_2)_6-$ | $-CH_2CH_2-$ | -COOH | 93 |

Les thioamides suivant l'invention conviennent aux emplois habituels des thioamides. Ils peuvent être utilisés pour la protection des plantes contre des parasites. Dans une application intéressante, le thioamide sert de collecteur de minerais en flottation, comme illustré par l'exemple 21 ci-après.

**Exemple 21**

Des essais de flottation, dans une cellule HALLIMOND, sont effectués à la manière classique, décrite dans le brevet français publié sous le n°2 429 617, page 3, lignes 26 - 40.

Le collecteur employé est le N-bis(hydroxy-2 éthyl) dodécane-thioamide de l'exemple 19 donné plus haut. Il est utilisé en solution éthanolique à 1 %, à raison de 100 g de collecteur par tonne de minerai. Voici les % des minéraux flottés à différents pH.

| pH | Galène | Blende | Pyrite |
|---|---|---|---|
| 5,5 | 93 % | 82 % | 40 % |
| 7,02 | 91 % | 80 % | 56 % |
| 9,01 | 84 % | 13 % | 23 % |
| 10,5 | 82 % | 12 % | 11 % |

On voit que, jusqu'à pH 7 les résultats avec la galène sont remarquables, et sur la blende assez bons. A partir de pH 9, la séparation de ces deux minéraux est très aisée. Le thioamide se montre de même utile à la séparation de la pyrite. Ce collecteur convient également dans le cas de la chalcopyrite.

**Revendications**

1. Procédé de flottation de minerais, dans lequel un thioamide est employé en tant que collecteur, caractérisé en ce que ce thioamide porte un second groupe fonctionnel et répond à la formule

```
R-C-NH-R"Z  ou  R-C-N(R"Z)2
  ||              ||
  S               S
```

où

R est un alkyle, alkényle, cycloalkyle, cycloalkényle ou aryle, éventuellement substitué,

R" est un alkylène, alkénylène, cycloalkylène, cycloalkénylène ou arylène, éventuellement substitué comme R ou différemment, et

Z, le second groupe fonctionnel en plus de celui du groupe amide, est -OH, $-NH_2$ ou -COOH, dont les H peuvent être substitués par un radical hydrocarboné, un groupe d'amine cyclique ou un groupe $-HC(OCH_3)_2$.

2. Procédé suivant la revendication 1, caractérisé en ce que R" est une chaîne $-(CH_2)_n-$ n étant de 1 à 18.

3. Procédé suivant la revendication 1 ou 2, caractérisé en ce que R est un alkyle linéaire en $C_7$ à $C_{11}$, n est 1 à 6 et Z est -OH, $-NH_2$, $-OCH_3$, $-N(CH_3)_2$ ou -COOH.

4. Procédé suivant la revendication 1 ou 2, caractérisé en ce que R est un phényle, chloro-phényle, tolyle ou méthoxyphényle, n est 1 à 6 et Z est -OH, $-NH_2$, $-N(CH_3)_2$,$-HC(OCH_3)_2$ ou pyridyle.

5. Thioamide de formule

```
R-C-NH-R"Z  ou  R-C-N(R"Z)2
  ||              ||
  S               S
```

où R et R" sont des groupes hydrocarbonés et Z un groupe fonctionnel OH ou COOH, dont le H peut être substitué, un groupe $-HC(OCH_3)_2$ ou bien $NH_2$, caractérisé en ce que R est une chaîne alkylique en $C_7$ à $C_{11}$, R" étant une chaîne $-(CH_2)_n-$ avec n = 1 à 3 ou bien

```
- CH
  |
  CH3
```

6. Thioamide suivant la revendication 5, caractérisé en ce que Z est $-OCH_3$.

7. Thioamide suivant la revendication 5, caractérisé en ce que Z est

```
  OCH3
  |
-CH
  |
  OCH3
```

8. Thioamide suivant la revendication 7, caractérisé en ce que R est un groupe chlorophényle et R" est $-CH_2-$.

9. Procédé de préparation d'un thioamide, suivant une des revendications 5 à 8, qui consiste à faire réagir un ester mono- ou di-thioïque, correspondant à cet amide, au sein d'un solvant, avec une amine porteuse d'un second groupement fonctionnel, caractérisé en ce que l'amine utilisée est choisie parmi les amines

```
                                                  OCH3
                                                  |
NH2-R"-OH, NH2-R"-COOH, NH2-R"-NH2 ou NH2-R"-CH,
                                                  |
                                                  OCH3
```

dans lesquelles R" est une chaîne $-(CH_2)_n-$ avec n = 1 à 3 ou bien un groupe

- CH-,
|
$CH_3$

l'atome de H dans l'OH ou -COOH pouvant être substitué par un alkyle, et en ce que la réaction est conduite à une température comprise entre 0° et 50° C.

10. Procédé suivant la revendication 9, dans lequel la réaction est réalisée au sein d'un hydrocarbure ou hydrocarbure chloré, caractérisé en ce qu'elle a lieu entre 0° et 25° C.

**Patentansprüche**

1. Verfahren zur Flotation von Mineralien, bei dem ein Thioamid als Kollektor verwandt wird, dadurch gekennzeichnet, daß dieses Thioamid eine zweite funktionelle Gruppe trägt und der Formel

R-C-NH-R"Z oder R-C-N(R"Z)$_2$
‖ ‖
S S

entspricht, worin

R ein Alkyl, Alkenyl, Cycloalkyl, Cycloalkenyl oder Aryl, gegebenenfalls substituiert, ist,

R" ein Alkylen, Alkenylen, Cycloalkylen, Cycloalkenylen oder Arylen, gegebenenfalls wie R oder verschieden davon substituiert, ist und

Z, die zweite funktionelle Gruppe zusätzlich zur Amidgruppe, -OH, $-NH_2$ oder -COOH ist, wovon die H durch einen Kohlenwasserstoffrest, eine cyclische Aminogruppe oder eine Gruppe $-HC(OCH_3)_2$ ersetzt sein können.

2. Verfahren nach Anspruch 1, dadurch gekennzeichnet, daß R" eine Kette $-(CH_2)_n-$ ist, worin n 1 bis 18 ist.

3. Verfahren nach Anspruch 1 oder 2, dadurch gekennzeichnet, daß R ein lineares $C_7$-$C_{11}$-Alkyl ist, n 1 bis 6 ist und Z -OH, $-NH_2$, $-OCH_3$, $-N(CH_3)_2$ oder -COOH ist.

4. Verfahren nach Anspruch 1 oder 2, dadurch gekennzeichnet, daß R ein Phenyl, Chlorphenyl, Tolyl oder Methoxyphenyl ist, n 1 bis 6 ist und Z -OH, $-NH_2$, $-N(CH_3)_2$, $-HC(OCH_3)_2$ oder Pyridyl ist.

5. Thioamid der Formel

R-C-NH-R"Z oder R-C-N(R"Z)$_2$,
‖ ‖
S S

worin R und R" Kohlenwasserstoffgruppen sind und Z eine funktionelle Gruppe OH oder COOH, wovon das H substituiert sein kann, eine Gruppe $-HC(OCH_3)_2$ oder auch $-NH_2$ ist, dadurch gekennzeichnet, daß R eine $C_7$-$C_{11}$-Alkylkette ist, R" eine Kette $-(CH_2)_n-$ ist, worin n = 1 bis 3, oder
- CH- ist.
|
$CH_3$

6. Thioamid nach Anspruch 5, dadurch gekennzeichnet, daß Z $-OCH_3$ ist.

7. Thioamid nach Anspruch 5, dadurch gekennzeichnet, daß Z

$OCH_3$
|
-CH
|
$OCH_3$ ist.

8. Thioamid nach Anspruch 7, dadurch gekennzeichnet, daß R eine Chlorphenylgruppe ist und R" $-CH_2-$ ist.

9. Verfahren zur Herstellung eines Thioamids nach einem der Ansprüche 5 bis 8 durch Umsetzung eines Mono- oder Dithiosäureesters, der diesem Amid entspricht, mit einem Amin, das eine zweite funktionelle Gruppe trägt, in einem Lösungsmittel, dadurch gekennzeichnet, daß das verwendete Amin ausgewählt wird aus den Aminen

$OCH_3$
|
$NH_2$-R"-OH, $NH_2$-R"-COOH, $NH_2$-R"-$NH_2$ oder $NH_2$-R"-CH,
|
$OCH_3$

in welchen R'' eine Kette $-(CH_2)_n-$ mit n = 1 bis 3 oder auch eine Gruppe

- CH
  |
  $CH_3$

ist, wobei das H-Atom in OH oder -COOH durch ein Alkyl ersetzt sein kann, und daß die Reaktion bei einer Temperatur zwischen 0 und 50° C durchgeführt wird.

10. Verfahren nach Anspruch 9, worin die Reaktion in einem Kohlenwasserstoff oder chlorierten Kohlenwasserstoff durchgeführt wird, dadurch gekennzeichnet, daß sie bei 0 bis 25° C stattfindet.

## Claims

1. Process of flotation of minerals, in which a thioamide is employed as the collector, characterised in that this thioamide carries a second functional group and corresponds to the formula

R-C-NH-R''Z or R-C-N(R''Z)$_2$
  ||          ||
  S          S

where

R is an alkyl, alkenyl, cycloalkyl, cycloalkenyl or aryl, possibly substituted,

R'' is an alkylene, alkenylene, cycloalkylene, cycloalkenylene or arylene, possibly substituted like R or differently, and

Z, the second functional group in addition to that of the amide group, is -OH, $-NH_2$ or -COOH, the H's of which can be substituted by a hydrocarbon radical, a cyclic amine group or a group $-HC(OCH_3)_2$.

2. Process according to claim 1, characterised in that R'' is a chain $-(CH_2)_n-$, n being from 1 to 18.

3. Process according to claim 1 or 2, characterised in that R is a $C_7$ to $C_{11}$ linear alkyl, n is 1 to 6 and Z is -OH, $-NH_2$, $-OCH_3$, $-N(CH_3)_2$ or -COOH.

4. Process according to claim 1 or 2, characterised in that R is a phenyl, chloro-phenyl, tolyl or methoxyphenyl, n is 1 to 6 and Z is -OH, $-NH_2$, $-N(CH_3)_2$, $-HC(OCH_3)_2$ or pyridyl.

5. Thioamide of the formula

R-C-NH-R''Z or R-C-N(R''Z)$_2$
  ||          ||
  S          S

where R and R'' are hydrocarbon groups and Z is a functional OH or COOH group, the H of which can be substituted, a group $-HC(OCH_3)_2$ or $NH_2$, characterised in that R is a $C_7$ to $C_{11}$ alkyl chain, R'' being a chain $-(CH_2)_n-$ with n = 1 to 3 or

- CH-.
  |
  $CH_3$

6. Thioamide according to claim 5, characterised in that Z is $-OCH_3$.

7. Thioamide according to claim 5, characterised in that Z is

  $OCH_3$
  |
-CH
  |
  $OCH_3$

8. Thioamide according to claim 7, characterised in that R a chlorophenyl group and R'' is $-CH_2-$.

9. Process of preparation of a thioamide, according to any of claims 5 to 8, which consists in reacting a mono- or di-thioic ester, corresponding to this amide, in a solvent with an amine carrying a second functional group, characterised in that the amine utilised is selected from the amines

                                                                  $OCH_3$
                                                                  |
$NH_2$-R''-OH, $NH_2$-R''-COOH, $NH_2$-R''-$NH_2$ or $NH_2$-R''-CH,
                                                                  |
                                                                  $OCH_3$

in which R'' is a chain $-(CH_2)_n-$ with n = 1 to 3 or a group

$$\begin{array}{l} -\ CH-, \\ \quad | \\ \quad CH_3 \end{array}$$

the H atom in the OH or the -COOH possibly being substituted by an alkyl and in that the reaction is carried out at a temperature in the range from 0° to 50°C.

10. Process according to claim 9, in which the reaction is carried out in a hydrocarbon or chlorinated hydrocarbon, characterised in that it takes place between 0° and 25°C.